(19) Europäisches Patentamt
European Patent Office
Office européen des brevets

(11) Veröffentlichungsnummer: **0 640 600 A1**

# (12) EUROPÄISCHE PATENTANMELDUNG

(21) Anmeldenummer: **94112809.2**

(22) Anmeldetag: **17.08.94**

(51) Int. Cl.6: **C07D 413/04**, C07D 265/36, C07D 279/16

(30) Priorität: **30.08.93 DE 4329096**

(43) Veröffentlichungstag der Anmeldung: **01.03.95 Patentblatt 95/09**

(84) Benannte Vertragsstaaten: **BE CH DE ES FR GB IT LI NL**

(71) Anmelder: **BAYER AG**
**D-51368 Leverkusen (DE)**

(72) Erfinder: **Schallner, Otto, Dr.**
**Noldeweg 22**
**D-40789 Monheim (DE)**
Erfinder: **Santel, Hans-Joachim, Dr,**
**Grünstrasse 9a**
**D-51371 Leverkusen (DE)**
Erfinder: **Dollinger, Markus, Dr.**
**Burscheider Strasse 154b**
**D-51381 Leverkusen (DE)**

(54) **Heterocyclylbenzoheterocyclen.**

(57) Die Erfindung betrifft neue Heterocyclylbenzoheterocyclen der allgemeinen Formel (I)

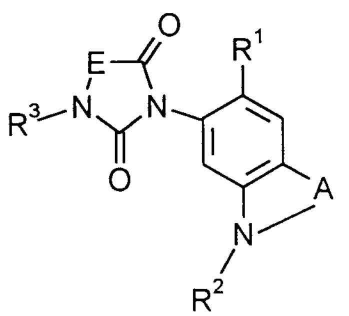

(I)

in welcher
$R^1$, $R^2$, $R^3$, A und E die in der Beschreibung genannten Bedeutungen haben,
Verfahren zu ihrer Herstellung, ihre Verwendung als Herbizide sowie neue Zwischenprodukte.

EP 0 640 600 A1

Die Enfindung betrifft neue Heterocyclylbenzoheterocyclen, Verfahren zu ihrer Herstellung und ihre Verwendung als Herbizide, sowie neue Zwischenprodukte.

Es ist bereits bekannt, daß bestimmte Heterocyclylbenzoheterocyclen herbizide Eigenschaften aufweisen (vgl. EP-A 170191, EP-A 218972).

Diese vorbekannten Verbindungen haben bis heute jedoch keine besondere Bedeutung erlangt.

Es wurden nun die neuen Heterocyclylbenzoheterocyclen der allgemeinen Formel (I) gefunden,

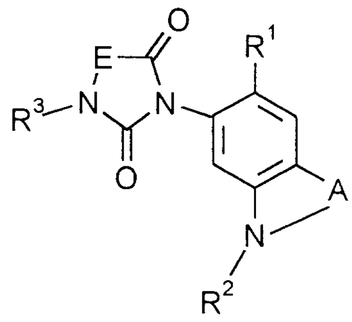

(I)

in welcher

| | |
|---|---|
| $R^1$ | für Wasserstoff oder Halogen steht, |
| $R^2$ | für Wasserstoff, Hydroxy oder für einen jeweils gegebenenfalls substituierten Rest der Reihe Alkyl, Alkenyl, Alkinyl, Cycloalkyl, Cycloalkylalkyl, Alkoxy, Alkenyloxy, Arylalkyl oder Arylalkyloxy steht, |
| $R^3$ | für Wasserstoff Cyano oder für einen jeweils gegebenenfalls substituierten Rest der Reihe Alkyl, Alkenyl oder Alkinyl steht, |
| A | für eine der nachstehenden Gruppierungen steht -O-CO-, -S-CO-, -O-C($R^4$,$R^5$)-CO-, -C($R^4$,$R^5$)-O-CO-, -C($R^4$,$R^5$)-CO--O-CO-C($R^4$,$R^5$)-, -C($R^4$,$R^5$)-C($R^4$,$R^5$)-, -N($R^6$)-C($R^4$,$R^5$)-CO-, |

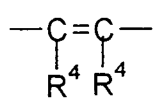

,

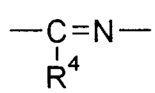

wobei

| | |
|---|---|
| $R^4$, $R^5$ und $R^6$ | gleich oder verschieden sind und einzeln für Wasserstoff oder Alkyl oder zusammen für Alkandiyl stehen und |
| E | für eine der nachstehenden Gruppierungen steht |

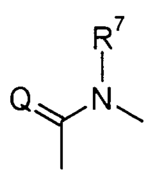

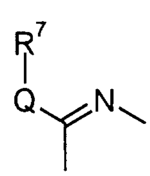

wobei

| | |
|---|---|
| Q | für Sauerstoff oder Schwefel steht und |
| $R^7$ | für Wasserstoff oder für gegebenenfalls substituiertes Alkyl steht. |

Die allgemeine Formel (I) steht also für die isomeren Verbindungen der nachstehenden allgemeinen Formeln (IA) und (IB)

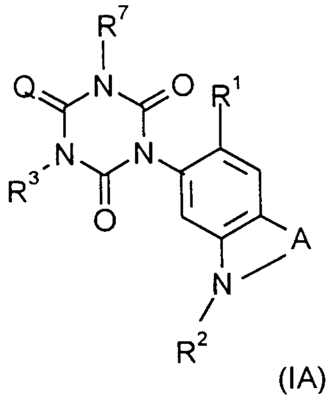

(IA)

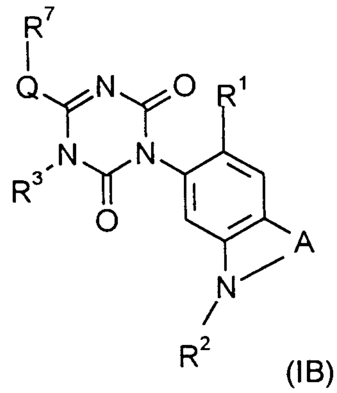

(IB)

Man erhält die neuen Heterocyclylbenzoheterocyclen der allgemeinen Formel (I), wenn man substituierte Ureidobenzoheterocyclen der allgemeinen Formel (II)

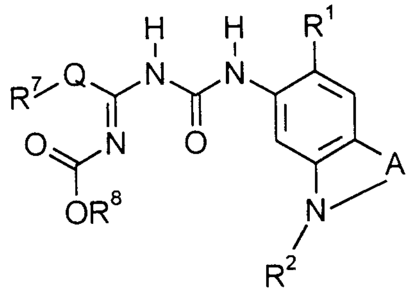

(II)

in welcher

$R^1$, $R^2$, $R^7$, A und Q die oben angegebenen Bedeutungen haben und

$R^8$ für Alkyl oder Aryl steht,

gegebenenfalls in Gegenwart eines Reaktionshilfsmittels und gegebenenfalls in Gegenwart eines Verdünnungsmittels cyclisiert

und die hierbei erhaltenen Heterocyclylbenzoheterocyclen der allgemeinen Formel (IB1)

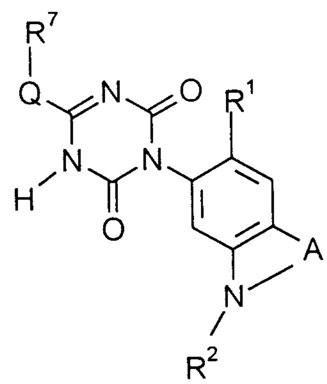

(IB1)

in welcher

$R^1$, $R^2$, $R^7$, A und Q die oben angegebenen Bedeutungen haben,

gegebenenfalls in einer anschließenden zweiten Reaktionsstufe mit Alkylierungsmitteln der Formel (III)

$X\text{-}R^{3-1}$ (III)

in welcher

$R^{3-1}$ für gegebenenfalls substituiertes Alkyl steht und

X für Halogen oder die Gruppierung $-O-SO_2-O-R^{3-1}$ steht,

oder mit einem Halogencyan, gegebenenfalls in Gegenwart eines Reaktionshilfsmittels und gegebenenfalls in Gegenwart eines Verdünnungsmittels umsetzt,

und wenn man gegebenenfalls die so erhaltenen Verbindungen der Formel (IB) gegebenenfalls in einer dritten Reaktionsstufe nach üblichen Methoden - wie beispielsweise durch thermische Isomerisierung - gegebenenfalls in Gegenwart eines Reaktionshilfmittels und gegebenenfalls in Gegenwart eines Verdünnungsmittels in Verbindungen der Formel (IA) umwandelt.

Die neuen Heterocyclylbenzoheterocyclen der allgemeinen Formel (I) zeichnen sich durch starke herbizide Wirksamkeit aus.

In den Definitionen sind die gesättigten oder ungesättigten Kohlenwasserstoffketten, wie Alkyl, Alkenyl oder Alkinyl, auch in Verbindung mit Heteroatomen, wie in Alkoxy, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Gegenstand der Erfindung sind vorzugsweise Verbindungen der Formel (I), in welcher

$R^1$ für Wasserstoff, Fluor, Chlor oder Brom steht,

$R^2$ für Wasserstoff, Hydroxy, für gegebenenfalls durch Halogen, Cyano, $C_1$-$C_4$-Alkoxy, $C_1$-$C_4$-Alkyl-carbonyl oder $C_1$-$C_4$-Alkoxy-carbonyl substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Halogen oder $C_1$-$C_4$-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, für jeweils gegebenenfalls durch Halogen substituiertes Alkoxy oder Alkenyloxy, oder für jeweils gegebenenfalls durch Halogen, Cyano, $C_1$-$C_4$-Alkyl, $C_1$-$C_4$-Halogenalkyl, $C_1$-$C_4$-Alkoxy oder $C_1$-$C_4$-Halogenalkoxy substituiertes Benzyl oder Benzyloxy steht,

$R^3$ Wasserstoff, für gegebenenfalls durch Halogen, Cyano, $C_1$-$C_4$-Alkoxy, $C_1$-$C_4$-Alkylcarbonyl oder $C_1$-$C_4$-Alkoxy-carbonyl substituiertes Alkyl mit 1 bis 6 Kohlenstoffatomen oder für jeweils gegebenenfalls durch Halogen substituiertes Alkenyl oder Alkinyl mit jeweils 2 bis 6 Kohlenstoffatomen steht,

A für eine der nachstehenden Gruppierungen steht

$-O-CO-$, $-S-CO-$, $-O-C(R^4,R^5)-CO-$, $-C(R^4,R^5)-O-CO-$, $-C(R^4,R^5)-C(R^4,R^5)-$-$C(R^4)=C(R^4)-$, $-C(R^4,R^5)-CO$, $-N(R^6)-C(R^4,R^5)-CO-$, $-C(R^4)=N-$, $-O-CO-C(R^4,R^5)-$

wobei

$R^4$ und $R^5$ gleich oder verschieden sind und einzeln für Wasserstoff oder Alkyl mit 1 bis 6 Kohlenstoffatomen oder zusammen für Alkandiyl mit 2 bis 6 Kohlenstoffatomen stehen,

$R^6$ für Wasserstoff oder Alkyl mit 1 bis 6 Kohlenstoffatomen steht, und

E für eine der nachstehenden Gruppierungen steht

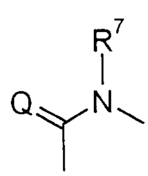

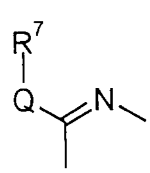

wobei

Q für Sauerstoff oder Schwefel steht und

$R^7$ für Wasserstoff oder für gegebenenfalls substituiertes Alkyl mit 1 bis 6 Kohlenstoffatomen steht.

Die Erfindung betrifft insbesondere Verbindungen der Formel (I), in welcher

$R^1$ für Wasserstoff, Fluor oder Chlor steht,

$R^2$ für Wasserstoff steht,

$R^2$ weiterhin für jeweils gegebenenfalls durch Fluor, Chlor, Cyano, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl steht,

$R^2$ weiterhin für jeweils gegebenenfalls durch Fluor, Chlor oder Brom substituiertes Allyl, 1-Methyl-2-propen-1-yl, 1,1-Dimethyl-2-propen-1-yl, 2-Buten-1-yl, Propargyl, 1-Methyl-2-propin-1-yl, 1,1-Dimethyl-2-propin-1-yl oder 2-Butin-1-yl, für jeweils gegebenenfalls durch Fluor, Chlor, Brom, Methyl, Ethyl, n- oder i-Propyl, n-, i-, s- oder t-Butyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,

$R^2$ weiterhin für jeweils gegebenenfalls durch Fluor oder Chlor substituiertes Methoxy, Ethoxy, n- oder i-Propoxy, n-, i-, s- oder t-Butoxy, Allyloxy oder 2-Buten-1-yl-oxy steht, oder

$R^2$ weiterhin für jeweils gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Benzyl oder Benzyloxy steht,

$R^3$ für Wasserstoff steht,

$R^3$ weiterhin für gegebenenfalls durch Fluor, Chlor, Cyano, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl steht, oder

$R^3$ weiterhin für jeweils gegebenenfalls durch Fluor, Chlor oder Brom substituiertes Allyl, 1-Methyl-2-propen-1-yl, 1,1-Dimethyl-2-propen-1-yl, 2-Buten-1-yl, Propargyl, 1-Methyl-2-propin-1-yl, 1,1-Dimethyl-2-propin-1-yl oder 2-Butin-1-yl steht,

A für eine der nachstehenden Gruppierungen steht

-O-CO-, -S-CO-, -O-C($R^4$,$R^5$)-CO-, -C($R^4$,$R^5$)-O-CO-, -O-CO-C($R^4$,$R^5$)-, -C($R^4$,$R^5$)-C($R^4$,$R^5$)-, -C($R^4$) = C($R^4$)-, -C($R^4$,$R^5$)-CO-, -N($R^6$)-C($R^4$,$R^5$)-CO-, -C($R^4$) = N-

wobei

$R^4$ und $R^5$ gleich oder verschieden sind und einzeln für Wasserstoff, Methyl, Ethyl, n- oder i-Propyl oder zusammen für Ethan-1,2-diyl stehen,

$R^6$ für Wasserstoff, Methyl, Ethyl, n- oder i-Propyl steht, und

E für eine der nachstehenden Gruppierungen steht

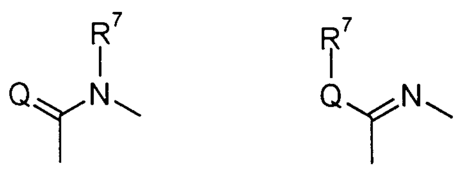

wobei

Q für Sauerstoff oder Schwefel steht und

$R^7$ für Wasserstoff, Methyl, Ethyl, n- oder i-Propyl, n-, i- oder s-Butyl steht.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Beispiele für die möglichen Kombinationen der oben definierten Gruppierungen $R^1$, $R^2$, $R^3$, $R^7$, A und Q sind nachstehend aufgeführt.:

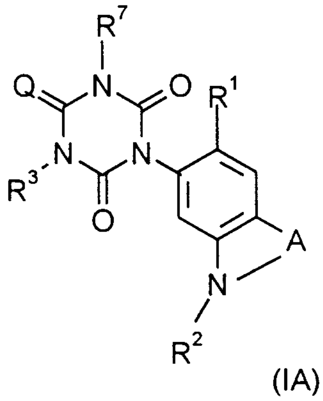

(IA)

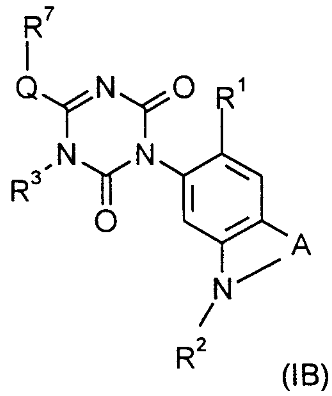

(IB)

| $R^1$ | $R^2$ | $R^3$ | $R^7$ | A | Q |
|---|---|---|---|---|---|
| H | $-CH_2C{\equiv}CH$ | $CH_3$ | $C_2H_5$ | -S-CO- | O |
| H | $-CH_2C{\equiv}CH$ | $CH_3$ | $C_2H_5$ | -O-CO- | O |
| H | $-CH_2C{\equiv}CH$ | $CH_3$ | $C_2H_5$ | $-CH_2$-O-CO- | O |
| H | $-CH_2C{\equiv}CH$ | $CH_3$ | $C_2H_5$ | $-C(CH_3)_2$-CO- | O |
| H | $-CH_2C{\equiv}CH$ | $CH_3$ | $C_2H_5$ | $-O-CH(CH_3)$-CO- | O |
| H | $-CH_2C{\equiv}CH$ | $CH_3$ | $C_2H_5$ | 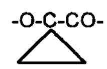 | O |
| H | $-CH_2C{\equiv}CH$ | $CH_3$ | $C_2H_5$ | 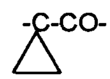 | O |
| H | $-CH_2C{\equiv}CH$ | $CH_3$ | $C_2H_5$ | -CH=CH- | O |
| H | $-CH_2C{\equiv}CH$ | $CH_3$ | $C_2H_5$ | -CH=N- | O |
| H | $-CH_2C{\equiv}CH$ | $CH_3$ | $C_2H_5$ | $-N(CH_3)-CH_2$-CO | O |
| H | $-CH_2C{\equiv}CH$ | $CH_3$ | $C_2H_5$ | $-N(CH_3)$-CO- | O |
| H | $-CH_2C{\equiv}CH$ | $CH_3$ | $C_2H_5$ | $-O-COCH_2-$ | O |
| H | $-CO-CH_3$ | $CH_3$ | $C_2H_5$ | $-O-COCH_2-$ | O |
| H | $-CH_2C{\equiv}CH$ | $CH_3$ | $C_2H_5$ | $-O-C(CH_3)_2$-CO- | O |
| H | $-CO-CH_3$ | $CH_3$ | $C_2H_5$ | $-CH_2-CH_2-$ | O |
| H | $-CH_2C{\equiv}CH$ | $CH_3$ | $C_2H_5$ | $-CH(CH_3)-CH_2-$ | O |
| H | $-CH_2C{\equiv}CH$ | $CH_3$ | $C_2H_5$ | $-OCH_2$-CO- | O |
| H | $-CH_2C{\equiv}CH$ | $CH_3$ | $CH_3$ | -S-CO- | S |
| H | $-CH_2C{\equiv}CH$ | $CH_3$ | $CH_3$ | -O-CO- | S |
| H | $-CH_2C{\equiv}CH$ | $CH_3$ | $CH_3$ | $-CH_2$-O-CO- | S |
| H | $-CH_2C{\equiv}CH$ | $CH_3$ | $CH_3$ | $-C(CH_3)_2$-CO- | S |
| H | $-CH_2C{\equiv}CH$ | $CH_3$ | $CH_3$ | $-O-CH(CH_3)$-CO- | S |
| H | $-CH_2C{\equiv}CH$ | $CH_3$ | $CH_3$ | 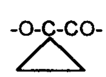 | S |
| H | $-CH_2C{\equiv}CH$ | $CH_3$ | $CH_3$ | 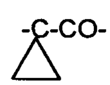 | S |

| | | | | | |
|---|---|---|---|---|---|
| H | $-CH_2C\equiv CH$ | $CH_3$ | $CH_3$ | $-CH=CH-$ | S |
| H | $-CH_2C\equiv CH$ | $CH_3$ | $CH_3$ | $-CH=N-$ | S |
| H | $-CH_2C\equiv CH$ | $CH_3$ | $CH_3$ | $-N(CH_3)-CH_2-CO$ | S |
| H | $-CH_2C\equiv CH$ | $CH_3$ | $CH_3$ | $-N(CH_3)-CO-$ | S |
| H | $-CH_2C\equiv CH$ | $CH_3$ | $CH_3$ | $-O-COCH_2-$ | S |
| H | $-CO-CH_3$ | $CH_3$ | $CH_3$ | $-O-CO-CH_2-$ | S |
| H | $-CH_2C\equiv H$ | $CH_3$ | $CH_3$ | $-O-C(CH_3)_2-CO-$ | S |
| H | $-CO-CH_3$ | $CH_3$ | $CH_3$ | $-CH_2-CH_2-$ | S |
| H | $-CH_2C\equiv CH$ | $CH_3$ | $CH_3$ | $-CH(CH_3)-CH_2-$ | S |
| F | $-CH_2C\equiv CH$ | $CH_3$ | $C_2H_5$ | $-S-CO-$ | O |
| F | $-CH_2C\equiv CH$ | $CH_3$ | $C_2H_5$ | $-O-CO-$ | O |
| F | $-CH_2C\equiv CH$ | $CH_3$ | $C_2H_5$ | $-CH_2-O-CO-$ | O |
| F | $-CH_2C\equiv CH$ | $CH_3$ | $C_2H_5$ | $-C(CH_3)_2-CO-$ | O |
| F | $-CH_2C\equiv CH$ | $CH_3$ | $C_2H_5$ | $-O-CH(CH_3)-CO-$ | O |
| F | $-CH_2C\equiv CH$ | $CH_3$ | $C_2H_5$ | 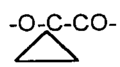 | O |
| F | $-CH_2C\equiv CH$ | $CH_3$ | $C_2H_5$ | 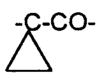 | O |
| F | $-CH_2C\equiv CH$ | $CH_3$ | $C_2H_5$ | $-CH=CH-$ | O |
| F | $-CH_2C\equiv CH$ | $CH_3$ | $C_2H_5$ | $-CH=N-$ | O |
| F | $-CH_2C\equiv CH$ | $CH_3$ | $C_2H_5$ | $-N(CH_3)-CH_2-CO$ | O |
| F | $-CH_2C\equiv CH$ | $CH_3$ | $C_2H_5$ | $-N(CH_3)-CO-$ | O |
| F | $-CH_2C\equiv CH$ | $CH_3$ | $C_2H_5$ | $-O-COCH_2-$ | O |
| F | $-CO-CH_3$ | $CH_3$ | $C_2H_5$ | $-O-COCH_2-$ | O |
| F | $-CH_2C\equiv CH$ | $CH_3$ | $C_2H_5$ | $-O-C(CH_3)_2-CO-$ | O |
| F | $-CO-CH_3$ | $CH_3$ | $C_2H_5$ | $-CH_2-CH_2-$ | O |
| F | $-CH_2C\equiv CH$ | $CH_3$ | $CH_3$ | $-CH(CH_3)-CH_2-$ | O |
| F | $-CH_2C\equiv CH$ | $CH_3$ | $CH_3$ | $-S-CO-$ | S |
| F | $-CH_2C\equiv CH$ | $CH_3$ | $CH_3$ | $-O-CO-$ | S |
| F | $-CH_2C\equiv CH$ | $CH_3$ | $CH_3$ | $-CH_2-O-CO-$ | S |

| | | | | | |
|---|---|---|---|---|---|
| F | $-CH_2C\equiv CH$ | $CH_3$ | $CH_3$ | $-C(CH_3)_2-CO-$ | S |
| F | $-CH_2C\equiv CH$ | $CH_3$ | $CH_3$ | $-O-CH(CH_3)-CO-$ | S |
| F | $-CH_2C\equiv CH$ | $CH_3$ | $CH_3$ | 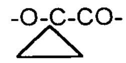 | S |
| F | $-CH_2C\equiv CH$ | $CH_3$ | $CH_3$ | 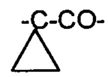 | S |
| F | $-CH_2C\equiv CH$ | $CH_3$ | $CH_3$ | $-CH=CH-$ | S |
| F | $-CH_2C\equiv CH$ | $CH_3$ | $CH_3$ | $-CH=N-$ | S |
| F | $-CH_2C\equiv CH$ | $CH_3$ | $CH_3$ | $-N(CH_3)-CH_2-CO$ | S |
| F | $-CH_2C\equiv CH$ | $CH_3$ | $CH_3$ | $-N(CH_3)-CO-$ | S |
| F | $-CH_2C\equiv CH$ | $CH_3$ | $CH_3$ | $-O-COCH_2-$ | S |
| F | $-CO-CH_3$ | $CH_3$ | $CH_3$ | $-O-CO-CH_2-$ | S |
| F | $-CH_2C\equiv CH$ | $CH_3$ | $CH_3$ | $-O-C(CH_3)_2-CO-$ | S |
| F | $-CO-CH_3$ | $CH_3$ | $CH_3$ | $-CH_2-CH_2-$ | S |
| F | $-CH_2C\equiv CH$ | $CH_3$ | $CH_3$ | $-CH(CH_3)-CH_2-$ | S |
| H | $-CH_2C\equiv CH$ | $CH_3$ | $CH_3$ | $-O-CH_2-CO-$ | S |
| F | $-CH_2CN$ | $CH_3$ | $CH_3$ | $-S-CO-$ | S |
| F | $-CH_2CN$ | $CH_3$ | $CH_3$ | $-O-CO-$ | S |
| F | $-CH_2CN$ | $CH_3$ | $CH_3$ | $-CH_2-O-CO-$ | S |
| F | $-CH_2CN$ | $CH_3$ | $CH_3$ | $-C(CH_3)_2-CO-$ | S |
| F | $-CH_2CN$ | $CH_3$ | $CH_3$ | $-O-CH(CH_3)-CO-$ | S |
| F | $-CH_2CN$ | $CH_3$ | $CH_3$ | 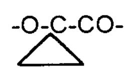 | S |
| F | $-CH_2CN$ | $CH_3$ | $CH_3$ | 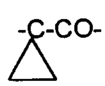 | S |
| F | $-CH_2CN$ | $CH_3$ | $CH_3$ | $-CH=CH-$ | S |
| F | $-CH_2CN$ | $CH_3$ | $CH_3$ | $-CH=N-$ | S |
| F | $-CH_2CN$ | $CH_3$ | $CH_3$ | $-N(CH_3)-CH_2-CO$ | S |
| F | $-CH_2CN$ | $CH_3$ | $CH_3$ | $-N(CH_3)-CO-$ | S |

| | | | | | |
|---|---|---|---|---|---|
| F | $-CH_2CN$ | $CH_3$ | $CH_3$ | $-O-COCH_2-$ | S |
| F | $-CH_2CN$ | $CH_3$ | $CH_3$ | $-O-C(CH_3)_2-CO-$ | S |
| F | $-CH_2CN$ | $CH_3$ | $CH_3$ | $-CH(CH_3)-CH_2-$ | S |
| F | $-CH_2CN$ | $CH_3$ | $CH_3$ | $-OCH_2-CO-$ | S |
| F | $-CH_2CN$ | $CH_3$ | $C_2H_5$ | $-S-CO-$ | O |
| F | $-CH_2CN$ | $CH_3$ | $C_2H_5$ | $-O-CO-$ | O |
| F | $-CH_2CN$ | $CH_3$ | $C_2H_5$ | $-CH_2-O-CO-$ | O |
| F | $-CH_2CN$ | $CH_3$ | $C_2H_5$ | $-C(CH_3)_2-CO-$ | O |
| F | $-CH_2CN$ | $CH_3$ | $C_2H_5$ | $-O-CH(CH_3)-CO-$ | O |
| F | $-CH_2CN$ | $CH_3$ | $C_2H_5$ | 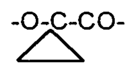 | O |
| F | $-CH_2CN$ | $CH_3$ | $C_2H_5$ | 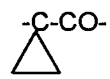 | O |
| F | $-CH_2CN$ | $CH_3$ | $C_2H_5$ | $-CH=CH-$ | O |
| F | $-CH_2CN$ | $CH_3$ | $C_2H_5$ | $-CH=N-$ | O |
| F | $-CH_2CN$ | $CH_3$ | $C_2H_5$ | $-N(CH_3)-CH_2-CO$ | O |
| F | $-CH_2CN$ | $CH_3$ | $C_2H_5$ | $-N(CH_3)-CO-$ | O |
| F | $-CH_2CN$ | $CH_3$ | $C_2H_5$ | $-O-COCH_2-$ | O |
| F | $-CH_2CN$ | $CH_3$ | $C_2H_5$ | $-O-C(CH_3)_2-CO-$ | O |
| F | $-CH_2CN$ | $CH_3$ | $C_2H_5$ | $-CH(CH_3)-CH_2-$ | O |
| F | $-CH_2CN$ | $CH_3$ | $C_2H_5$ | $-OCH_2-CO-$ | O |
| F | $-CH_2C\equiv CH$ | CN | $C_2H_5$ | $-S-CO-$ | O |
| F | $-CH_2C\equiv CH$ | CN | $C_2H_5$ | $-O-CO-$ | O |
| F | $-CH_2C\equiv CH$ | CN | $C_2H_5$ | $-CH_2-O-CO-$ | O |
| F | $-CH_2C\equiv CH$ | CN | $C_2H_5$ | $-C(CH_3)_2-CO-$ | O |
| F | $-CH_2C\equiv CH$ | CN | $C_2H_5$ | $-O-CH(CH_3)-CO-$ | O |
| F | $-CH_2C\equiv CH$ | CN | $C_2H_5$ | 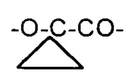 | O |

| | | | | | |
|---|---|---|---|---|---|
| F | $-CH_2C\equiv CH$ | CN | $C_2H_5$ | 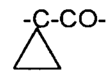 | O |
| F | $-CH_2C\equiv CH$ | CN | $C_2H_5$ | -CH=CH- | O |
| F | $-CH_2C\equiv CH$ | CN | $C_2H_5$ | -CH=N- | O |
| F | $-CH_2C\equiv CH$ | CN | $C_2H_5$ | $-N(CH_3)-CH_2-CO$ | O |
| F | $-CH_2C\equiv H$ | CN | $C_2H_5$ | $-N(CH_3)-CO-$ | O |
| F | $-CH_2C\equiv CH$ | CN | $C_2H_5$ | $-O-COCH_2-$ | O |
| F | $-CO-CH_3$ | CN | $C_2H_5$ | $-O-CO-CH_2-$ | O |
| F | $-CH_2C\equiv H$ | CN | $C_2H_5$ | $-O-C(CH_3)_2-CO-$ | O |
| F | $-CO-CH_3$ | CN | $C_2H_5$ | $-CH_2-CH_2-$ | O |
| F | $-CH_2C\equiv CH$ | CN | $C_2H_5$ | $-CH(CH_3)-CH_2-$ | O |
| F | $-CH_2C\equiv CH$ | CN | $C_2H_5$ | $-O-CH_2-CO-$ | O |
| F | $-CH_2C\equiv CH$ | CN | $CH_3$ | -S-CO- | S |
| F | $-CH_2C\equiv CH$ | CN | $CH_3$ | -O-CO- | S |
| F | $-CH_2C\equiv CH$ | CN | $CH_3$ | $-CH_2-O-CO-$ | S |
| F | $-CH_2C\equiv CH$ | CN | $CH_3$ | $-C(CH_3)_2-CO-$ | S |
| F | $-CH_2C\equiv CH$ | CN | $CH_3$ | $-O-CH(CH_3)-CO-$ | S |
| F | $-CH_2C\equiv CH$ | CN | $CH_3$ | 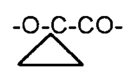 | S |
| F | $-CH_2C\equiv CH$ | CN | $CH_3$ | 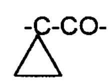 | S |
| F | $-CH_2C\equiv CH$ | CN | $CH_3$ | -CH=CH- | S |
| F | $-CH_2C\equiv CH$ | CN | $CH_3$ | -CH=N- | S |
| F | $-CH_2C\equiv CH$ | CN | $CH_3$ | $-N(CH_3)-CH_2-CO$ | S |
| F | $-CH_2C\equiv CH$ | CN | $CH_3$ | $-N(CH_3)-CO-$ | S |
| F | $-CH_2C\equiv CH$ | CN | $CH_3$ | $-O-COCH_2-$ | S |
| F | $-CO-CH_3$ | CN | $CH_3$ | $-O-CO-CH_2-$ | S |
| F | $-CH_2C\equiv CH$ | CN | $CH_3$ | $-O-C(CH_3)_2-CO-$ | S |
| F | $-CO-CH_3$ | CN | $CH_3$ | $-CH_2-CH_2-$ | S |

| | | | | | |
|---|---|---|---|---|---|
| F | $-CH_2C\equiv CH$ | CN | $CH_3$ | $-CH(CH_3)-CH_2-$ | S |
| F | $-CH_2C\equiv CH$ | CN | $CH_3$ | $-O-CH_2-CO-$ | S |
| F | $CH_2-CH=CH_2$ | $CH_3$ | $CH_3$ | -S-CO- | S |
| F | $CH_2-CH=CH_2$ | $CH_3$ | $CH_3$ | -O-CO- | S |
| F | $CH_2-CH=CH_2$ | $CH_3$ | $CH_3$ | $-CH_2-O-CO-$ | S |
| F | $CH_2-CH=CH_2$ | $CH_3$ | $CH_3$ | $-C(CH_3)_2-CO-$ | S |
| F | $CH_2-CH=CH_2$ | $CH_3$ | $CH_3$ | $-O-CH(CH_3)-CO-$ | S |
| F | $CH_2-CH=CH_2$ | $CH_3$ | $CH_3$ | 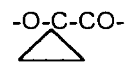 | S |
| F | $CH_2-CH=CH_2$ | $CH_3$ | $CH_3$ | 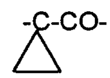 | S |
| F | $CH_2-CH=CH_2$ | $CH_3$ | $CH_3$ | -CH=CH- | S |
| F | $CH_2-CH=CH_2$ | $CH_3$ | $CH_3$ | -CH=N- | S |
| F | $CH_2-CH=CH_2$ | $CH_3$ | $CH_3$ | $-N(CH_3)-CH_2-CO$ | S |
| F | $CH_2-CH=CH_2$ | $CH_3$ | $CH_3$ | $-N(CH_3)-CO-$ | S |
| F | $CH_2-CH=CH_2$ | $CH_3$ | $CH_3$ | $-O-COCH_2-$ | S |
| F | $CH_2-CH=CH_2$ | $CH_3$ | $CH_3$ | $-O-C(CH_3)_2-CO-$ | S |
| F | $CH_2-CH=CH_2$ | $CH_3$ | $CH_3$ | $-CH(CH_3)-CH_2-$ | S |
| F | $CH_2-CH=CH_2$ | $CH_3$ | $CH_3$ | $-O-CH_2-CO-$ | S |
| F | $CH_2-CH=CH_2$ | $CH_3$ | $C_2H_5$ | -S-CO- | O |
| F | $CH_2-CH=CH_2$ | $CH_3$ | $C_2H_5$ | -O-CO- | O |
| F | $CH_2-CH=CH_2$ | $CH_3$ | $C_2H_5$ | $-CH_2-O-CO-$ | O |
| F | $CH_2-CH=CH_2$ | $CH_3$ | $C_2H_5$ | $-C(CH_3)_2-CO-$ | O |
| F | $CH_2-CH=CH_2$ | $CH_3$ | $C_2H_5$ | $-O-CH(CH_3)-CO-$ | O |
| F | $CH_2-CH=CH_2$ | $CH_3$ | $C_2H_5$ | 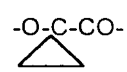 | O |
| F | $CH_2-CH=CH_2$ | $CH_3$ | $C_2H_5$ | 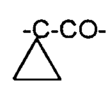 | O |
| F | $CH_2-CH=CH_2$ | $CH_3$ | $C_2H_5$ | -CH=CH- | O |

| | | | | | |
|---|---|---|---|---|---|
| F | $CH_2$-CH=$CH_2$ | $CH_3$ | $C_2H_5$ | -CH=N- | O |
| F | $CH_2$-CH=$CH_2$ | $CH_3$ | $C_2H_5$ | -N($CH_3$)-$CH_2$-CO | O |
| F | $CH_2$-CH=$CH_2$ | $CH_3$ | $C_2H_5$ | -N($CH_3$)-CO- | O |
| F | $CH_2$-CH=$CH_2$ | $CH_3$ | $C_2H_5$ | -O-CO$CH_2$- | O |
| F | $CH_2$-CH=$CH_2$ | $CH_3$ | $C_2H_5$ | -O-C($CH_3$)$_2$-CO- | O |
| F | $CH_2$-CH=$CH_2$ | $CH_3$ | $C_2H_5$ | -CH($CH_3$)-$CH_2$- | O |
| F | $CH_2$-CH=$CH_2$ | $CH_3$ | $C_2H_5$ | -O-$CH_2$-CO- | O |
| F | -$CH_2C\equiv CH$ | $CHF_2$ | $C_2H_5$ | -S-CO- | O |
| F | -$CH_2C\equiv CH$ | $CHF_2$ | $C_2H_5$ | -O-CO- | O |
| F | -$CH_2C\equiv CH$ | $CHF_2$ | $C_2H_5$ | -$CH_2$-O-CO- | O |
| F | -$CH_2C\equiv CH$ | $CHF_2$ | $C_2H_5$ | -C($CH_3$)$_2$-CO- | O |
| F | -$CH_2C\equiv CH$ | $CHF_2$ | $C_2H_5$ | -O-CH($CH_3$)-CO- | O |
| F | -$CH_2C\equiv CH$ | $CHF_2$ | $C_2H_5$ | 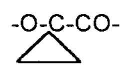 | O |
| F | -$CH_2C\equiv CH$ | $CHF_2$ | $C_2H_5$ | 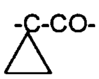 | O |
| F | -$CH_2C\equiv CH$ | $CHF_2$ | $C_2H_5$ | -CH=CH- | O |
| F | -$CH_2C\equiv CH$ | $CHF_2$ | $C_2H_5$ | -CH=N- | O |
| F | -$CH_2C\equiv H$ | $CHF_2$ | $C_2H_5$ | -N($CH_3$)-$CH_2$-CO | O |
| F | -$CH_2C\equiv CH$ | $CHF_2$ | $C_2H_5$ | -N($CH_3$)-CO- | O |
| F | -$CH_2C\equiv CH$ | $CHF_2$ | $C_2H_5$ | -O-CO$CH_2$- | O |
| F | -CO-$CH_3$ | $CHF_2$ | $C_2H_5$ | -O-CO-$CH_2$- | O |
| F | -$CH_2C\equiv CH$ | $CHF_2$ | $C_2H_5$ | -O-C($CH_3$)$_2$-CO- | O |
| F | -CO-$CH_3$ | $CHF_2$ | $C_2H_5$ | -$CH_2$-$CH_2$- | O |
| F | -$CH_2C\equiv CH$ | $CHF_2$ | $C_2H_5$ | -CH($CH_3$)-$CH_2$- | O |
| F | -$CH_2C\equiv CH$ | $CHF_2$ | $C_2H_5$ | -O-$CH_2$-CO- | O |
| F | -$CH_2C\equiv CH$ | $CH_3$ | $C_2H_5$ | -S-CO- | S |
| F | -$CH_2C\equiv CH$ | $CH_3$ | $C_2H_5$ | -O-CO- | S |
| F | -$CH_2C\equiv CH$ | $CH_3$ | $C_2H_5$ | -$CH_2$-O-CO- | S |
| F | -$CH_2C\equiv CH$ | $CH_3$ | $C_2H_5$ | -C($CH_3$)$_2$-CO- | S |

| | | | | | |
|---|---|---|---|---|---|
| F | $-CH_2C{\equiv}CH$ | $CH_3$ | $C_2H_5$ | $-O-CH(CH_3)-CO-$ | S |
| F | $-CH_2C{\equiv}CH$ | $CH_3$ | $C_2H_5$ | 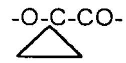 | S |
| F | $-CH_2C{\equiv}CH$ | $CH_3$ | $C_2H_5$ | 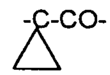 | S |
| F | $-CH_2C{\equiv}CH$ | $CH_3$ | $C_2H_5$ | $-CH=CH-$ | S |
| F | $-CH_2C{\equiv}CH$ | $CH_3$ | $C_2H_5$ | $-CH=N-$ | S |
| F | $-CH_2C{\equiv}CH$ | $CH_3$ | $C_2H_5$ | $-N(CH_3)-CH_2-CO$ | S |
| F | $-CH_2C{\equiv}CH$ | $CH_3$ | $C_2H_5$ | $-N(CH_3)-CO-$ | S |
| F | $-CH_2C{\equiv}CH$ | $CH_3$ | $C_2H_5$ | $-O-COCH_2-$ | S |
| F | $-CO-CH_3$ | $CH_3$ | $C_2H_5$ | $-O-CO-CH_2-$ | S |
| F | $-CH_2C{\equiv}CH$ | $CH_3$ | $C_2H_5$ | $-O-C(CH_3)_2-CO-$ | S |
| F | $-CO-CH_3$ | $CH_3$ | $C_2H_5$ | $-CH_2-CH_2-$ | S |
| F | $-CH_2C{\equiv}CH$ | $CH_3$ | $C_2H_5$ | $-CH(CH_3)-CH_2-$ | S |
| F | $-CH_2C{\equiv}CH$ | $CH_3$ | $C_2H_5$ | $-O-CH_2-CO-$ | S |
| F | $CH_2-CH=CH_2$ | $CH_3$ | $CH_3$ | $-S-CO-$ | S |
| F | $CH_2-CH=CH_2$ | $CH_3$ | $CH_3$ | $-O-CO-$ | S |
| F | $CH_2-CH=CH_2$ | $CH_3$ | $CH_3$ | $-CH_2-O-CO-$ | S |
| F | $CH_2-CH=CH_2$ | $CH_3$ | $CH_3$ | $-C(CH_3)_2-CO-$ | S |
| F | $CH_2-CH=CH_2$ | $CH_3$ | $CH_3$ | $-O-CH(CH_3)-CO-$ | S |
| F | $CH_2-CH=CH_2$ | $CH_3$ | $CH_3$ | 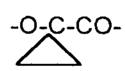 | S |
| F | $CH_2-CH=CH_2$ | $CH_3$ | $CH_3$ | 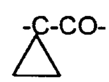 | S |
| F | $CH_2-CH=CH_2$ | $CH_3$ | $CH_3$ | $-CH=CH-$ | S |
| F | $CH_2-CH=CH_2$ | $CH_3$ | $CH_3$ | $-CH=N-$ | S |
| F | $CH_2-CH=CH_2$ | $CH_3$ | $CH_3$ | $-N(CH_3)-CH_2-CO$ | S |
| F | $CH_2-CH=CH_2$ | $CH_3$ | $CH_3$ | $-N(CH_3)-CO-$ | S |
| F | $CH_2-CH=CH_2$ | $CH_3$ | $CH_3$ | $-O-COCH_2-$ | S |

| | | | | | |
|---|---|---|---|---|---|
| F | $CH_2$-CH=$CH_2$ | $CH_3$ | $CH_3$ | -O-C($CH_3$)$_2$-CO- | S |
| F | $CH_2$-CH=$CH_2$ | $CH_3$ | $CH_3$ | -CH($CH_3$)-$CH_2$- | S |
| F | $CH_2$-CH=$CH_2$ | $CH_3$ | $CH_3$ | -O-$CH_2$-CO- | S |
| F | $CH_2$-CH=CHCl | $CH_3$ | $C_2H_5$ | -S-CO- | O |
| F | $CH_2$-CH=CHCl | $CH_3$ | $C_2H_5$ | -O-CO- | O |
| F | $CH_2$-CH=CHCl | $CH_3$ | $C_2H_5$ | -$CH_2$-O-CO- | O |
| F | $CH_2$-CH=CHCl | $CH_3$ | $C_2H_5$ | -C($CH_3$)$_2$-CO- | O |
| F | $CH_2$-CH=CHCl | $CH_3$ | $C_2H_5$ | -O-CH($CH_3$)-CO- | O |
| F | $CH_2$-CH=CHCl | $CH_3$ | $C_2H_5$ | 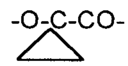 | O |
| F | $CH_2$-CH=CHCl | $CH_3$ | $C_2H_5$ | 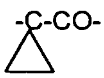 | O |
| F | $CH_2$-CH=CHCl | $CH_3$ | $C_2H_5$ | -CH=CH- | O |
| F | $CH_2$-CH=CHCl | $CH_3$ | $C_2H_5$ | -CH=N- | O |
| F | $CH_2$-CH=CHCl | $CH_3$ | $C_2H_5$ | -N($CH_3$)-$CH_2$-CO | O |
| F | $CH_2$-CH=CHCl | $CH_3$ | $C_2H_5$ | -N($CH_3$)-CO- | O |
| F | $CH_2$-CH=CHCl | $CH_3$ | $C_2H_5$ | -O-CO$CH_2$- | O |
| F | $CH_2$-CH=CHCl | $CH_3$ | $C_2H_5$ | -O-C($CH_3$)$_2$-CO- | O |
| F | $CH_2$-CH=CHCl | $CH_3$ | $C_2H_5$ | -CH($CH_3$)-$CH_2$- | O |
| F | $CH_2$-CH=CHCl | $CH_3$ | $C_2H_5$ | -O-$CH_2$-CO- | O |
| F | $-CH_2C\equiv CH$ | $CHF_2$ | $CH_3$ | -S-CO- | S |
| F | $-CH_2C\equiv CH$ | $CHF_2$ | $CH_3$ | -O-CO- | S |
| F | $-CH_2C\equiv CH$ | $CHF_2$ | $CH_3$ | -$CH_2$-O-CO- | S |
| F | $-CH_2C\equiv CH$ | $CHF_2$ | $CH_3$ | -C($CH_3$)$_2$-CO- | S |
| F | $-CH_2C\equiv CH$ | $CHF_2$ | $CH_3$ | -O-CH($CH_3$)-CO- | S |
| F | $-CH_2C\equiv CH$ | $CHF_2$ | $CH_3$ | 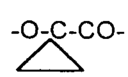 | S |
| F | $-CH_2C\equiv CH$ | $CHF_2$ | $CH_3$ | 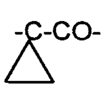 | S |

| | | | | | |
|---|---|---|---|---|---|
| F | $-CH_2C{\equiv}CH$ | $CHF_2$ | $CH_3$ | -CH=CH- | S |
| F | $-CH_2C{\equiv}CH$ | $CHF_2$ | $CH_3$ | -CH=N- | S |
| F | $-CH_2C{\equiv}CH$ | $CHF_2$ | $CH_3$ | $-N(CH_3)-CH_2-CO$ | S |
| F | $-CH_2C{\equiv}CH$ | $CHF_2$ | $CH_3$ | $-N(CH_3)-CO-$ | S |
| F | $-CH_2C{\equiv}CH$ | $CHF_2$ | $CH_3$ | $-O-COCH_2-$ | S |
| F | $-CO-CH_3$ | $CHF_2$ | $CH_3$ | $-O-CO-CH_2-$ | S |
| F | $-CH_2C{\equiv}CH$ | $CHF_2$ | $CH_3$ | $-O-C(CH_3)_2-CO-$ | S |
| F | $-CO-CH_3$ | $CHF_2$ | $CH_3$ | $-CH_2-CH_2-$ | S |
| F | $-CH_2C{\equiv}CH$ | $CHF_2$ | $CH_3$ | $-CH(CH_3)-CH_2-$ | S |
| F | $-CH_2C{\equiv}CH$ | $CHF_2$ | $CH_3$ | $-O-CH_2-CO-$ | S |
| H | $-CH_2C{\equiv}CH$ | CN | $C_2H_5$ | -S-CO- | O |
| H | $-CH_2C{\equiv}CH$ | CN | $C_2H_5$ | -O-CO- | O |
| H | $-CH_2C{\equiv}CH$ | CN | $C_2H_5$ | $-CH_2-O-CO-$ | O |
| H | $-CH_2C{\equiv}CH$ | CN | $C_2H_5$ | $-C(CH_3)_2-CO-$ | O |
| H | $-CH_2C{\equiv}CH$ | CN | $C_2H_5$ | $-O-CH(CH_3)-CO-$ | O |
| H | $-CH_2C{\equiv}CH$ | CN | $C_2H_5$ | 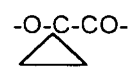 | O |
| H | $-CH_2C{\equiv}CH$ | CN | $C_2H_5$ | 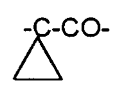 | O |
| H | $-CH_2C{\equiv}CH$ | CN | $C_2H_5$ | -CH=CH- | O |
| H | $-CH_2C{\equiv}CH$ | CN | $C_2H_5$ | -CH=N- | O |
| H | $-CH_2C{\equiv}CH$ | CN | $C_2H_5$ | $-N(CH_3)-CH_2-CO$ | O |
| H | $-CH_2C{\equiv}CH$ | CN | $C_2H_5$ | $-N(CH_3)-CO-$ | O |
| H | $-CH_2C{\equiv}CH$ | CN | $C_2H_5$ | $-O-COCH_2-$ | O |
| H | $-CO-CH_3$ | CN | $C_2H_5$ | $-O-CO-CH_2-$ | O |
| H | $-CH_2C{\equiv}CH$ | CN | $C_2H_5$ | $-O-C(CH_3)_2-CO-$ | O |
| H | $-CO-CH_3$ | CN | $C_2H_5$ | $-CH_2-CH_2-$ | O |
| H | $-CH_2C{\equiv}CH$ | CN | $C_2H_5$ | $-CH(CH_3)-CH_2-$ | O |
| H | $-CH_2C{\equiv}CH$ | CN | $C_2H_5$ | $-O-CH_2-CO-$ | O |

Verwendet man beispielsweise 6-Fluor-5-[3-(1-ethoxy-1-methoxycarbonyliminomethyl)-ureido]-3-methyl-benzthiazol-2-on als Ausgangsverbindung und Methyliodid als Alkylierungsmittel, so läßt sich der Reaktionsablauf des erfindungsgemäßen Verfahrens durch das folgende Formelschema darstellen:

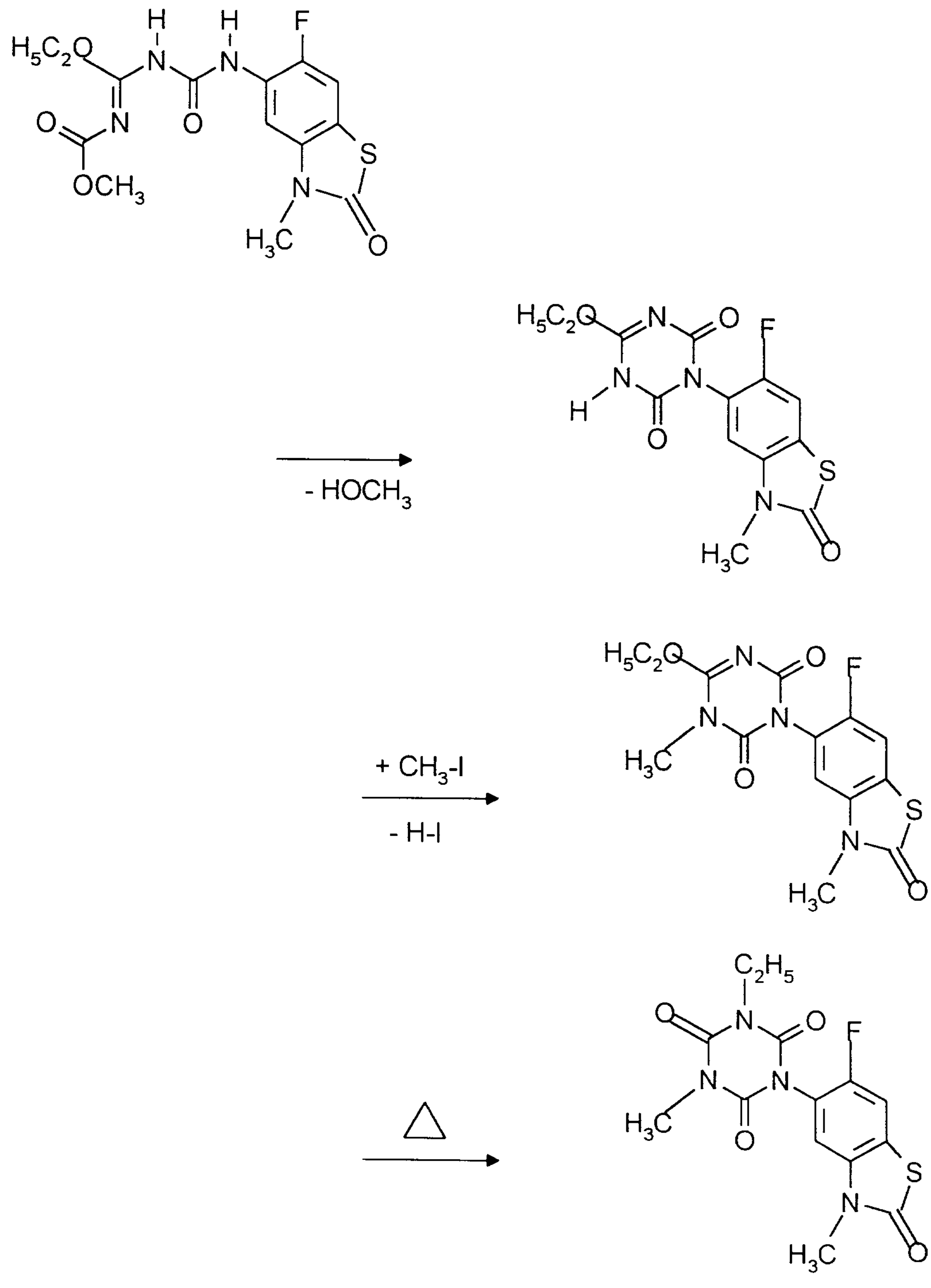


Die beim erfindungsgemäßen Verfahren zur Herstellung der Verbindungen der Formel (I) als Ausgangsstoffe zu verwendenden Ureidobenzoheterocyclen sind durch die Formel (II) allgemein definiert.

In der Formel (II) haben $R^1$, $R^2$, $R^7$, A und Q vorzugsweise bzw. insbesondere diejenigen Bedeutungen, die bereits oben im Zusammenhang mit der Beschreibung der erfindungsgemäßen Verbindungen der Formel (I) als bevorzugt bzw. als insbesondere bevorzugt für $R^1$, $R^2$, $R^7$, A und Q angegeben wurden.

Die als Ausgangsstoffe benötigten Ureidobenzoheterocyclen der Formel (II) sind noch nicht aus der Literatur bekannt; sie sind als neue Stoffe Gegenstand der vorliegenden Anmeldung.

Man erhält die neuen Verbindungen der Formel (II), wenn man Isocycanatobenzoheterocyclen der allgemeinen Formel (IV)

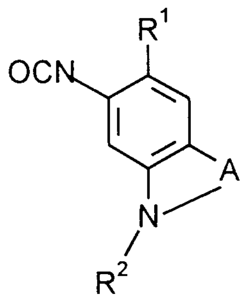

(IV)

in welcher

$R^1$, $R^2$ und A die oben angegebenen Bedeutungen haben,

mit Urethanen (Carbamaten) der Formel (V)

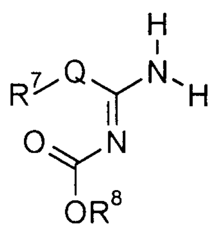

(V)

in welcher

$R^7$, $R^8$ und Q die oben angegebenen Bedeutungen haben,

gegebenenfalls in Gegenwart eines Verdünnungsmittels, wie z.B. Methylenchlorid, bei Temperaturen zwischen 0 °C und 50 °C umsetzt.

Die als Vorprodukte benötigten Isocyanatobenzoheterocyclen der Formel (IV) sind noch nicht aus der Literatur bekannt und sind als neue Stoffe Gegenstand der vorliegenden Anmeldung.

In der Formel (IV) haben $R^1$, $R^2$ und A vorzugsweise bzw. insbesondere diejenigen Bedeutungen, die bereits oben im Zusammenhang mit der Beschreibung der erfindungsgemäßen Verbindungen der Formel (I) als bevorzugt bzw. als insbesondere bevorzugt für $R^1$, $R^2$ und A angegeben wurden.

Die Verbindungen der Formel (IV) können im allgemeinen problemlos in einer vorgelagerten Umsetzung von entsprechenden Amino-heterocyclen der Formel (VI)

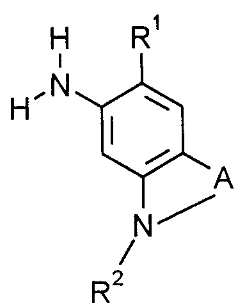

(VI)

in welcher

$R^1$, $R^2$ und A die oben angegebenen Bedeutungen haben,

mit Phosgen oder Diphosgen (Chlorameisensäure-trichlormethylester) auf übliche Weise hergestellt und ohne Zwischenisolierung mit den Urethanen der Formel (V) umgesetzt werden (vergleiche die Herstellungsbeispiele).

Die Aminoheterocyclen der Formel (VI) sind bekannt und/oder können nach an sich bekannten Verfahren hergestellt werden (vgl. EP-A 170191, EP-A 218972).

Die weiter als Vorprodukte benötigten Urethane (Carbamate) der Formel (V) sind bekannt und/oder können nach an sich bekannten Verfahren hergestellt werden (vgl. DE-OS 2933889; Liebigs Ann. Chem.

1985, 2363-2370).

Die zur Durchführung des erfindungsgemäßen Verfahrens gegebenenfalls weiterhin als Ausgangsstoffe zu verwendenden Alkylierungsmittel sind durch die Formel (III) allgemein definiert. In dieser Formel (III) steht $R^{3-1}$ vorzugsweise für gegebenenfalls durch Halogen, Cyano, $C_1$-$C_4$-Alkoxy, $C_1$-$C_4$-Alkyl-carbonyl oder $C_1$-$C_4$-Alkoxy-carbonyl substituiertes geradkettiges oder verzweigtes Alkyl mit 1 bis 6 Kohlenstoffatomen oder für jeweils gegebenenfalls durch Halogen substituiertes geradkettiges oder verzweigtes Alkenyl oder Alkinyl mit jeweils 2 bis 6 Kohlenstoffatomen; insbesondere für gegebenenfalls durch Fluor, Chlor, Cyano, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl, oder für jeweils gegebenenfalls durch Fluor, Chlor oder Brom substituiertes Allyl, 1-Methyl-2-propen-1-yl, 1,1-Dimethyl-2-propen-1-yl, 2-Buten-1-yl, Propargyl, 1-Methyl-2-propin-1-yl, 1,1-Dimethyl-2-propin-1-yl oder 2-Butin-1-yl;

X steht vorzugsweise für Chlor, Brom, Iod, Methoxysulfonyloxy oder Ethoxysulfonyloxy.

Die Alkylierungsmittel der Formel (III) sind bekannte organische Synthesechemikalien.

Als Verdünnungsmittel zur Durchführung der 1. Stufe des erfindungsgemäßen Verfahrens kommen alle üblichen organischen oder anorganischen Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methylisobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Alkohole, wie Methanol, Ethanol, n-oder i-Propanol oder n-, i-, s- oder t-Butanol.

Die 1. Stufe des erfindungsgemäßen Verfahrens kann gegebenenfalls in Gegenwart eines geeigneten basischen Reaktionshilfsmittels durchgeführt werden. Als solche kommen alle üblichen anorganischen oder organischen Basen infrage. Hierzu gehören beispielsweise Erdalkali- oder Alkalimetallhydroxide, wie Natriumhydroxid, Kaliumhydroxid, Calciumhydroxid oder auch Ammoniumhydroxid, Alkalimetall(hydrogen)-carbonate, wie Natrium(hydrogen)carbonat, Kalium(hydrogen)carbonat oder Ammoniumcarbonat, Alkali- oder Erdalkalimetallacetate, wie Natriumacetat, Kaliumacetat, Calciumacetat oder Ammoniumacetat, Alkalimetall-alkoholate, wie Natrium- oder Kalium-methylat, Natrium- oder Kaliumethylat, Natrium- oder Kalium-tert-butylat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 1. Stufe des erfindungsgemäßen Verfahrens in einem größeren Bereich variiert werden. Im allgemeinen arbeitet man bei Temperaturen zwischen 0 °C und 150 °C, vorzugsweise bei Temperaturen zwischen 20 °C und 120 °C.

Die 1. Stufe des erfindungsgemäßen Verfahren wird üblicherweise unter Normaldruck durchgeführt. Es ist jedoch auch möglich unter erhöhtem oder vermindertem Druck zu arbeiten.

Zur Durchführung der 1. Stufe des erfindungsgemäßen Verfahrens setzt man pro Mol Ureidobenzoheterocyclus der Formel (II) im allgemeinen 0,01 bis 2,0 Mol, vorzugsweise 0,1 bis 1,0 Mol Base als Reaktionshilfsmittel ein. Die Reaktionsdurchführung, Aufarbeitung und Isolierung der Reaktionsprodukte erfolgt in Analogie zu bekannten Verfahren (vgl. die Herstellungsbeispiele).

Als Verdünnungsmittel zur Durchführung der 2. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder - diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Die 2. Stufe des erfindungsgemäßen Verfahrens wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Animoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die 2. Stufe des erfindungsgemäßen Verfahrens kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}/C_{15}$-alkylammoniumchlorid, Trimethyl-$C_{13}/C_{15}$-alkylammoniumbromid, Dibenzyl-dimethyl-ammoniummethylsulfat, Dimethyl-$C_{12}/C_{14}$-alkyl-benzylammoniumchlorid, Dimethyl-$C_{12}/C_{14}$-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der Durchführung der 2. Stufe des erfindungsgemäßen Verfahrens in einem größeren Bereich variiert werden. Im allgemeinen arbeitet man bei Temperaturen zwischen -20 °C und +120 °C, vorzugsweise bei Temperaturen zwischen 0 °C und 80 °C.

Die 2. Stufe des erfindungsgemäßen Verfahrens wird üblicherweise unter Normaldruck durchgeführt. Es ist jedoch auch möglich unter erhöhtem oder vermindertem Druck zu arbeiten.

Zur Durchführung der 2. Stufe des erfindungsgemäßen Verfahrens setzt man pro Mol Heterocyclylbenzoheterocyclus der Formel (IB1) im allgemeinen 1,0 bis 5,0 Mol, vorzugsweise 1,0 bis 2,5 Mol Alkylierungsmittel der Formel (III) oder alternativ Halogencyan, ein und verwendet dabei 1,0 bis 5,0 Mol, vorzugsweise 1,0 bis 2,5 Mol Base als Reaktionshilfsmittel und gegebenenfalls 0,001 bis 2,0 Mol, vorzugsweise 0,001 bis 1,0 Mol Phasentransfer-Katalysator.

Die Reaktionsdurchführung, Aufarbeitung und Isolierung der Reaktionsprodukte erfolgt in Analogie zu bekannten Verfahren (vgl. die Herstellungsbeispiele).

Als Verdünnungsmittel zur Durchführung der 3. Stufe des erfindungsgemäßen Verfahrens kommen übliche inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder - diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Die 3. Stufe des erfindungsgemäßen Verfahrens kann gegebenenfalls auch in Gegenwart eines geeigneten basischen Reaktionshilfsmittels durchgeführt werden. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 3. Stufe des erfindungsgemäßen Verfahrens in einem größeren Bereich variiert werden. Im allgemeinen arbeitet man bei Temperaturen zwischen 50 °C und 200 °C, vorzugsweise bei Temperaturen zwischen 80 °C und 150 °C.

Die 3. Stufe des erfindungsgemäßen Verfahren wird üblicherweise unter Normaldruck durchgeführt. Es ist jedoch auch möglich unter erhöhtem oder vermindertem Druck zu arbeiten.

Zur Durchführung der 3. Stufe des erfindungsgemäßen Verfahrens setzt man pro Mol Heterocyclylbenzoheterocyclus der Formel (IB) im allgemeinen 0,01 bis 2,0 Mol, vorzugsweise 0,1 bis 1,0 Mol Base als Reaktionshilfsmittel ein. Es ist jedoch auch möglich, die 3. Stufe des erfindungsgemäßen Verfahrens ohne den Zusatz eines basischen Reaktionshilfsmittels durchzuführen. Die Reaktionsdurchführung, Aufarbeitung und Isolierung der Reaktionsprodukte erfolgt in Analogie zu bekannten Verfahren (vgl. die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie ($^1H$-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Anibrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zu selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von

Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

Die Herstellung und die Verwendung der erfindungsgemäßen Wirkstoffe geht aus den nachfolgenden Beispielen hervor.

**Herstellungsbeispiele:**

Beispiel 1:

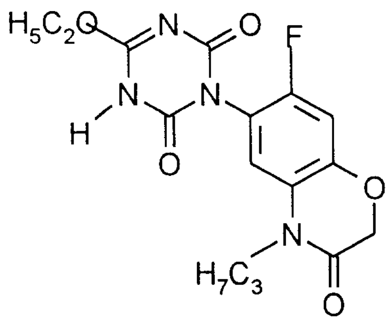

Zu einer Lösung von 8,0 g (0,02 Mol) 7-Fluor-6-[3-(1-ethoxy-1-methoxycarbonylimino-methyl)-ureido]-4-propyl-1,4-benzoxazin-3-on in 50 ml Methanol gibt man bei 20 °C 1,1 g (0,02 Mol) Natrium-methylat und erhitzt die Mischung 16 Stunden unter Rückfluß.

Dann wird das Lösungsmittel im Wasserstrahlvakuum abdestilliert, der Rückstand in Eiswasser gegeben und mit 2N-Salzsäure angesäuert. Das hierbei kristallin anfallende Produkt wird durch Abfiltrieren isoliert.

Man erhält 5,0 g (69% der Theorie) 3-(7-Fluor-3-oxo-4-propyl-1,4-benzoxazin-6-yl)-6-ethoxy-(1H,3H)-1,3,5-triazin-2,4-dion vom Schmelzpunkt 210 °C.

Beispiel 2

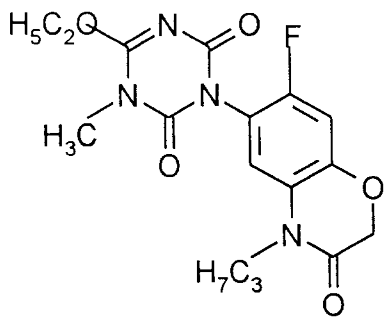

Zu einer Lösung von 1,8 g (5 mMol) 3-(7-Fluor-3-oxo-4-propyl-1,4-benzoxazin-6-yl)-6-ethoxy-(1H,3H)-1,3,5-triazin-2,4-dion in 30 ml Dimethylsulfoxid gibt man bei 20 °C nacheinander 1,4 g (10 mMol) Kaliumcarbonat und 0,7 g (5 mMol) Methyliodid und rührt das Gemisch 20 Stunden bei 20 °C.

Zur Aufarbeitung wird mit Wasser auf etwa das doppelte Volumen verdünnt, mit 2N-Salzsäure angesäuert und mit Methylenchlorid extrahiert. Das extrahierte Produkt wird säulenchromatographisch (Kieselgel, Methylenchlorid/Methanol, Vol. 40/1) gereinigt.

Man erhält 0,7 g (37% der Theorie) 3-(7-Fluor-3-oxo-4-propyl-1,4-benzoxazin-6-yl)-6-ethoxy-1-methyl-(1H,3H)-1,3,5-triazin-2,4-dion vom Schmelzpunkt 94 °C.

Beispiel 3

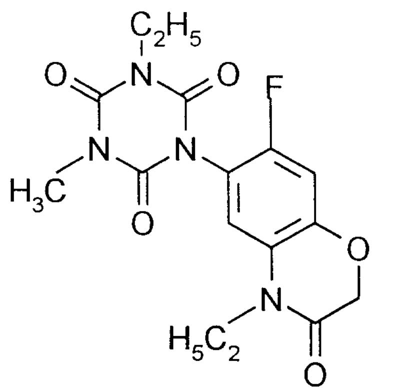

Eine Lösung von 0,5 g (1,3 mMol) 3-(7-Fluor-3-oxo-4-ethyl-1,4-benzoxazin-6-yl)-6-ethoxy-1-methyl-(1H,3H)-1,3,5-triazin-2,4-dion in 20 ml Toluol wird mit einem Tropfen Triethylamin versetzt und 16 Stunden unter Rückfluß erhitzt.

Anschließend werden die flüchtigen Komponenten im Wasserstrahlvakuum, dann im Ölpumpenvakuum sorgfältig abdestilliert. Man erhält 0,42 g (81.% der Theorie) 3-(7-Fluor-3-oxo-4-ethyl-1,4-benzoxazin-6-yl)1-ethyl-5-methyl-(1H,3H,5H)-1,3,5-triazin-2,4,6-trion vom Schmelzpunkt 91 °C.

Analog zu den Beispielen 1 bis 3 sowie entsprechend der allgemeinen Beschreibung des erfindungsgemäßen Herstellungsverfahrens können beispielsweise auch die in der nachstehenden Tabelle 1 aufgeführten Verbindungen der Formel (I) - bzw. der Formeln (IA) und (IB) - hergestellt werden.

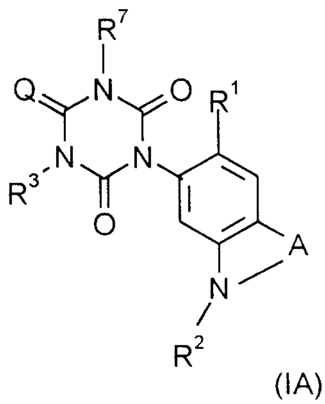
(IA)

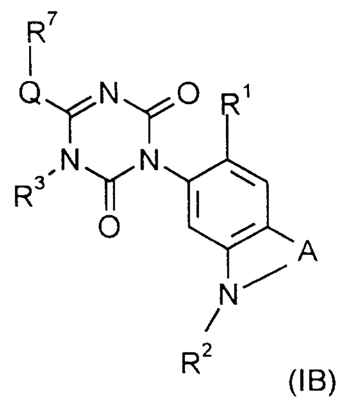
(IB)

Tabelle 1: Beispiele für die Verbindungen der Formel (I), d.h. (IA) oder (IB)

| Bsp.-Nr. | allg. Form. | $R^1$ | $R^2$ | $R^3$ | $R^7$ | A | Q | Schmelzpunkt |
|---|---|---|---|---|---|---|---|---|
| 4 | IB | F | $-CH_2-C{\equiv}CH$ | $CH_3$ | $C_2H_5$ | $-O-CH_2-CO-$ | O | 123°C |
| 5 | IB | F | $-CH_2-C{\equiv}CH$ | $CH_3$ | $CH_3$ | $-O-CH_2-CO-$ | S | >230°C |
| 6 | IA | F | $-CH_2-C{\equiv}CH$ | $CH_3$ | $CH_3$ | $-O-CH_2-CO-$ | O | 231°C |
| 7 | IA | F | $C_3H_7$-n | $CH_3$ | $CH_3$ | $-O-CH_2-CO-$ | O | 168°C |
| 8 | IB | F | $C_3H_7$-n | $CH_3$ | $CH_3$ | $-O-CH_2-CO-$ | S | 91°C |
| 9 | IB | F | $C_2H_5$ | $CH_3$ | $C_2H_5$ | $-O-CH_2-CO-$ | O | 201°C |
| 10 | IB | F | $-CH_2-C{\equiv}CH$ | H | $C_2H_5$ | $-O-CH_2-CO-$ | O | 123°C |
| 11 | IB | F | $-CH_2-C{\equiv}CH$ | H | $CH_3$ | $-O-CH_2-CO-$ | S | 149°C |
| 12 | IB | F | $-CH_2-C{\equiv}CH$ | H | $CH_3$ | $-O-CH_2-CO-$ | O | 131°C |
| 13 | IB | F | $C_3H_7$-n | H | $CH_3$ | $-O-CH_2-CO-$ | O | 162°C |
| 14 | IB | F | $C_3H_7$-n | H | $CH_3$ | $-O-CH_2-CO-$ | S | 64°C |
| 15 | IB | F | $C_2H_5$ | H | $C_2H_5$ | $-O-CH_2-CO-$ | O | 181°C |
| 16 | IB | F | $CH_2COOC_2H_5$ | H | $C_2H_5$ | $-O-CH_2-CO-$ | O | 184°C |
| 17 | IB | F | $-CH_2CH=CH_2$ | H | $C_2H_5$ | $-O-CH_2-CO-$ | O | 144°C |
| 18 | IB | F | $OC_2H_5$ | H | $C_2H_5$ | $-O-CH_2-CO-$ | O | |
| 19 | IB | F | $CH_2COOC_2H_5$ | $CH_3$ | $C_2H_5$ | $-O-CH_2-CO-$ | O | 54°C |
| 20 | IB | F | $CH_2-CH=CH_2$ | $CH_3$ | $C_2H_5$ | $-O-CH_2-CO-$ | O | 69°C |
| 21 | IB | F | $CH_2-C{\equiv}CH$ | H | $C_2H_5$ | $-OCH(CH_3)-CO-$ | O | 61°C |
| 22 | IB | F | $CH_2-C{\equiv}CH$ | $CH_3$ | $C_2H_5$ | $-O-CH(CH_3)-CO-$ | O | 75°C |
| 23 | IB | F | $CH_2-C{\equiv}CH$ | $CH_3$ | $CH_3$ | $-O-CH(CH_3)-CO-$ | S | 57°C |
| 24 | IA | F | $CH_2-C{\equiv}CH$ | $CH_3$ | $CH_3$ | $-O-CH(CH_3)-CO-$ | O | 64°C |
| 25 | IB | F | $C_3H_7$-n | $CH_3$ | $C_2H_5$ | $-O-CH(CH_3)-CO-$ | O | (Öl) |
| 26 | IA | F | $C_3H_7$-n | $CH_3$ | $CH_3$ | $-O-CH(CH_3)-CO-$ | O | 141°C |
| 27 | IA | F | $CH_2C{\equiv}CH$ | $CH_3$ | $C_2H_5$ | $-O-CH_2-CO-$ | O | 52°C |
| 28 | IB | F | $CH_2C{\equiv}CH$ | $C_2H_5$ | $CH_3$ | $-O-CH_2-CO-$ | O | 121°C |
| 29 | IB | F | $CH_2-CH=CH_2$ | $CH_3$ | $CH_3$ | $-O-CH_2-CO-$ | O | 72°C |
| 30 | IA | F | $CH_2-CH=CH_2$ | $C_2H_5$ | $CH_3$ | $-O-CH_2-CO-$ | O | (Öl) |
| 31 | IA | F | $CH_2-CH=CH_2$ | $CH_3$ | $C_2H_5$ | $-O-CH(CH_3)-CO-$ | O | 84°C |
| 32 | IA | F | $CH_2-CH=CH_2$ | $CH_3$ | $CH_3$ | $-O-CH_2-CO$ | O | 149°C |
| 33 | IB | H | $-CH_2-C{\equiv}CH$ | $C_2H_5$ | $CH_3$ | $-O-CH_2-CO-$ | O | 208°C |
| 34 | IB | H | $-CH_2-CH=CH_2$ | $C_2H_5$ | $CH_3$ | $-O-CH_2-CO-$ | O | 236°C |
| 35 | IB | H | $-CH_2-CH=CH_2$ | $CH_3$ | $C_2H_5$ | $-O-CH_2-CO-$ | O | 189°C |
| 36 | IB | H | $-CH_2-CH=CH_2$ | $CH_3$ | $CH_3$ | $-O-CH_2-CO-$ | S | > 230°C |
| 37 | IB | H | $-CH_2-C{\equiv}CH$ | $CH_3$ | $CH_3$ | $-O-CH_2-CO-$ | S | >230°C |
| 38 | IB | H | $-CH_2-C{\equiv}CH$ | $CH_3$ | $CH_3$ | $-O-CH_2-CO$ | O | >230°C |

Ausgangsstoffe der Formel (II):

Beispiel (II-1)

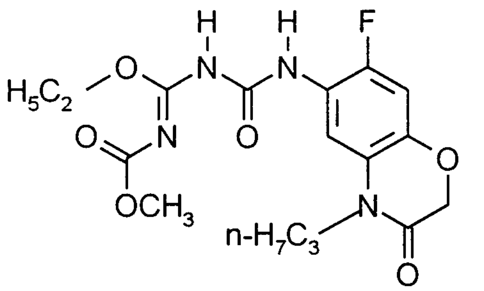

Zu einer Lösung von 4,4 g (0,02 Mol) 6-Amino-7-fluor-4-(n-propyl)-(2H)-1,4-benzoxazin-3-on in 50 ml Essigester gibt man bei 70 °C unter Rühren 4,0 g (0,02 Mol) Chlorameisensäuretrichlormethylester und erhitzt anschließend 2 Stunden auf Rückflußtemperatur. Anschließend wird das Lösungsmittel abdestilliert, der Rückstand in 100 ml Dichlormethan aufgenommen, mit 3,0 g (0,02 Mol) N-(1-Ethoxy-1-imino-methyl)-carbaminsäuremethylester versetzt und 16 Stunden bei 20 °C gerührt. Zur Aufarbeitung wird ausgefallener Niederschlag abfiltriert, das Filtrat im Vakuum eingeengt und der Rückstand mit Petrolether verrieben.

Man erhält 7,5 g (95% der Theorie) N-(7-Fluor-3-oxo-4-(n-propyl)-(2H)-1,4-benzoxazin-6-yl)-N'-(1-ethoxy-1-methoxcarbonylimino-methyl)-harnstoff mit dem Schmelzpunkt 84 °C.

Analog Beispiel (II-1) können beispielsweise auch die in der nachstehenden Tabelle 2 aufgeführten Verbindungen der Formel (II) hergestellt werden.

Tabelle 2

| Beispiele für die Ausgangsstoffe der Formel (II) | | | | | | | |
|---|---|---|---|---|---|---|---|
| Bsp.-Nr. | $R^1$ | $R^2$ | $R^7$ | $R^8$ | A | Q | Schmelzpunkt (°C) |
| II-2 | F | $-CH_2-C\equiv CH$ | $C_2H_5$ | $CH_3$ | $-O-CH_2-CO-$ | O | 120 |
| II-3 | F | $-CH_2-C\equiv CH$ | $CH_3$ | $CH_3$ | $-O-CH_2-CO-$ | S | 122 |
| II-4 | F | $-CH_2-C\equiv CH$ | $CH_3$ | $CH_3$ | $-O-CH_2-CO-$ | O | 127 |
| II-5 | F | $C_3H_7$-n | $CH_3$ | $CH_3$ | $-O-CH_2-CO-$ | O | 107 |
| II-6 | F | $C_3H_7$-n | $CH_3$ | $CH_3$ | $-O-CH_2-CO-$ | S | 131 |
| II-7 | F | $C_2H_5$ | $C_2H_5$ | $CH_3$ | $-O-CH_2-CO-$ | O | |
| II-8 | F | $-CH_2COOC_2H_5$ | $C_2H_5$ | $CH_3$ | $-O-CH_2-CO-$ | O | |
| II-9 | F | $-CH_2CH=CH_2$ | $C_2H_5$ | $CH_3$ | $-O-CH_2-CO-$ | O | |
| II-10 | F | $-OC_2H_5$ | $C_2H_5$ | $CH_3$ | $-O-CH_2-CO-$ | O | |
| II-11 | F | $-CH_2-C\equiv CH$ | $C_2H_5$ | $CH_3$ | $-O-CH(CH_3)-CO-$ | O | |

Anwendungsbeispiele:

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant.

Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

O % = keine Wirkung (wie unbehandelte Kontrolle)

100 % = totale Vernichtung

In diesem Test zeigt beispielsweise die Verbindung gemäß Herstellungsbeispiel 4 bei guter Verträglichkeit gegenüber Kulturpflanzen, wie z.B. Gerste, sehr starke Wirkung gegen Unkräuter.

Beispiel B

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

O % = keine Wirkung (wie unbehandelte Kontrolle)

100 % = totale Vernichtung

In diesem Test zeigen beispielsweise die Verbindungen gemäß den Herstellungsbeispielen 2, 4, 5, 6, 7 und 8 bei teilweise guter Verträglichkeit gegenüber Kulturpflanzen, wie z.B. Weizen und Soja, starke Wirkung gegen Unkräuter.

**Patentansprüche**

**1.** Heterocyclylbenzoheterocyclen der allgemeinen Formel (I)

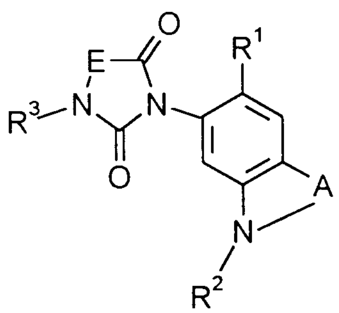

(I)

in welcher

$R^1$ für Wasserstoff oder Halogen steht,

$R^2$ für Wasserstoff, Hydroxy oder für einen jeweils gegebenenfalls substituierten Rest der Reihe Alkyl, Alkenyl, Alkinyl, Cycloalkyl, Cycloalkylalkyl, Alkoxy, Alkenyloxy, Arylalkyl oder Arylalkyloxy steht,

$R^3$ für Wasserstoff, Cyano oder für einen jeweils gegebenenfalls substituierten Rest der Reihe Alkyl, Alkenyl oder Alkinyl steht,

A für eine der nachstehenden Gruppierungen steht
-O-CO-, -S-CO-, -O-C($R^4$,$R^5$)-CO-, -C($R^4$,$R^5$)-O-CO-, -C($R^4$,$R^5$)-CO--O-CO-C-($R^4$,$R^5$)-, -C($R^4$,$R^5$)-C($R^4$,$R^5$)-, -N($R^6$)-C($R^4$,$R^5$)-CO-,

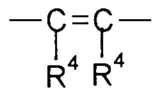 , 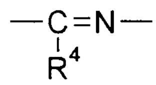

wobei

$R^4$, $R^5$ und $R^6$ gleich oder verschieden sind und einzeln für Wasserstoff oder Alkyl oder zusammen für Alkandiyl stehen und

E für eine der nachstehenden Gruppierungen steht

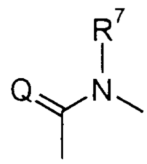 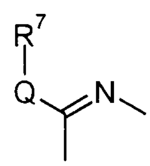

wobei

Q für Sauerstoff oder Schwefel steht und

$R^7$ für Wasserstoff oder für gegebenenfalls substituiertes Alkyl steht.

**2.** Heterocyclylbenzoheterocyclen der allgemeinen Formel (I) gemäß Anspruch 1, dadurch gekennzeichnet, daß

$R^1$ für Wasserstoff, Fluor, Chlor oder Brom steht,

$R^2$ für Wasserstoff, Hydroxy, für gegebenenfalls durch Halogen, Cyano, $C_1$-$C_4$-Alkoxy, $C_1$-$C_4$-Alkyl-carbonyl oder $C_1$-$C_4$-Alkoxy-carbonyl substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Halogen oder $C_1$-$C_4$-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, für jeweils gegebenenfalls durch Halogen substituiertes Alkoxy oder Alkenyloxy, oder für jeweils gegebenenfalls durch Halogen, Cyano, $C_1$-$C_4$-Alkyl, $C_1$-$C_4$-Halogenalkyl, $C_1$-$C_4$-Alkoxy oder $C_1$-$C_4$-Halogenalkoxy substituiertes Benzyl oder Benzyloxy steht,

$R^3$ Wasserstoff, für gegebenenfalls durch Halogen, Cyano, $C_1$-$C_4$-Alkoxy, $C_1$-$C_4$-Alkylcarbonyl oder $C_1$-$C_4$-Alkoxy-carbonyl substituiertes Alkyl mit 1 bis 6 Kohlenstoffatomen oder für jeweils gegebenenfalls durch Halogen substituiertes Alkenyl oder Alkinyl mit jeweils 2 bis 6 Kohlenstoffatomen steht,

A für eine der nachstehenden Gruppierungen steht
-O-CO-, -S-CO-, -O-C($R^4$,$R^5$)-CO-, -C($R^4$,$R^5$)-O-CO-, -C($R^4$,$R^5$)-C($R^4$,$R^5$)-, -C($R^4$)=C($R^4$)-, -C($R^4$,$R^5$)-CO-, -N($R^6$)-C($R^4$,$R^5$)-CO-, -C($R^4$)=N-, -O-CO-C($R^4$,$R^5$)-
wobei

$R^4$ und $R^5$ gleich oder verschieden sind und einzeln für Wasserstoff oder Alkyl mit 1 bis 6 Kohlenstoffatomen oder zusammen für Alkandiyl mit 2 bis 6 Kohlenstoffatomen stehen,

$R^6$ für Wasserstoff oder Alkyl mit 1 bis 6 Kohlenstoffatomen steht, und

E für eine der nachstehenden Gruppierungen steht

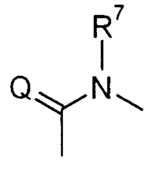 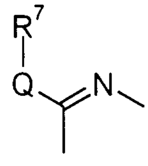

wobei

Q für Sauerstoff oder Schwefel steht und

$R^7$ für Wasserstoff oder für gegebenenfalls substituiertes Alkyl mit 1 bis 6 Kohlenstoffatomen steht.

**3.** Heterocyclylbenzoheterocyclen der allgemeinen Formel (I) gemäß Anspruch 1, dadurch gekennzeichnet, daß

$R^1$ für Wasserstoff, Fluor oder Chlor steht,

$R^2$ für Wasserstoff steht,

$R^2$ weiterhin für jeweils gegebenenfalls durch Fluor, Chlor, Cyano, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl steht,

$R^2$ weiterhin für jeweils gegebenenfalls durch Fluor, Chlor oder Brom substituiertes Allyl, 1-Methyl-2-propen-1-yl, 1,1-Dimethyl-2-propen-1-yl, 2-Buten-1-yl, Propargyl, 1-Methyl-2-propin-1-yl, 1,1-Dimethyl-2-propin-1-yl oder 2-Butin-1-yl, für jeweils gegebenenfalls durch Fluor, Chlor, Brom, Methyl, Ethyl, n- oder i-Propyl, n-, i-, s- oder t-Butyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,

$R^2$ weiterhin für jeweils gegebenenfalls durch Fluor oder Chlor substituiertes Methoxy, Ethoxy, n- oder i-Propoxy, n-, i-, s- oder t-Butoxy, Allyloxy oder 2-Buten-1-yl-oxy steht, oder

$R^2$ weiterhin für jeweils gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Benzyl oder Benzyloxy steht,

$R^3$ für Wasserstoff steht,

$R^3$ weiterhin für gegebenenfalls durch Fluor, Chlor, Cyano, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl steht, oder

$R^3$ weiterhin für jeweils gegebenenfalls durch Fluor, Chlor oder Brom substituiertes Allyl, 1-Methyl-2-propen-1-yl, 1,1-Dimethyl-2-propen-1-yl, 2-Buten-1-yl, Propargyl, 1-Methyl-2-propin-1-yl, 1,1-Dimethyl-2-propin-1-yl oder 2-Butin-1-yl steht,

A für eine der nachstehenden Gruppierungen steht

-O-CO-, -S-CO-, -O-C($R^4$,$R^5$)-CO-, -C($R^4$,$R^5$)-O-CO-, -O-CO-C($R^4$,$R^5$)-, -C($R^4$,$R^5$)-C($R^4$,$R^5$)-, -C($R^4$) = C($R^4$)-, -C($R^4$,$R^5$)-CO-, -N($R^6$)-C($R^4$,$R^5$)-CO-, -C($R^4$) = N-

wobei

$R^4$ und $R^5$ gleich oder verschieden sind und einzeln für Wasserstoff, Methyl, Ethyl, n- oder i-Propyl oder zusammen für Ethan-1,2-diyl stehen,

$R^6$ für Wasserstoff, Methyl, Ethyl, n- oder i-Propyl steht, und

E für eine der nachstehenden Gruppierungen steht

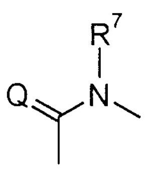

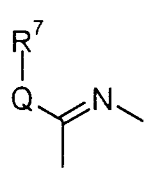

wobei

Q für Sauerstoff oder Schwefel steht und

$R^7$ für Wasserstoff, Methyl, Ethyl, n- oder i-Propyl, n-, i- oder s-Butyl steht.

**4.** Verfahren zur Herstellung von Heterocyclylbenzoheterocyclen der allgemeinen Formel (I)

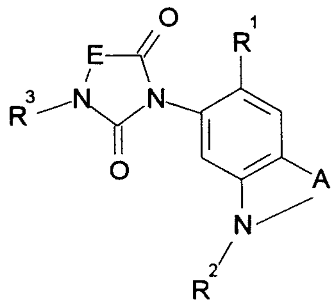

(I)

in welcher

$R^1$, $R^2$, $R^3$, A und E die in Anspruch 1 genannten Bedeutungen haben,

dadurch gekennzeichnet, daß man

substituierte Ureidobenzoheterocyclen der allgemeinen Formel (II)

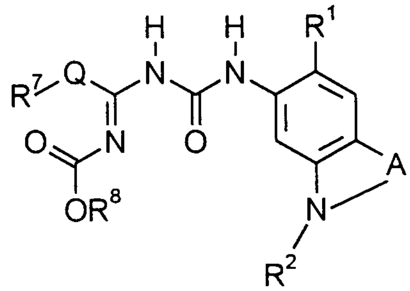

(II)

in welcher

$R^1$, $R^2$, $R^7$, A und Q die in Anspruch 1 genannten Bedeutungen haben und

$R^8$ für Alkyl oder Aryl steht,

gegebenenfalls in Gegenwart eines Reaktionshilfsmittels und gegebenenfalls in Gegenwart eines Verdünnungsmittels cyclisiert

und die hierbei erhaltenen Heterocyclylbenzoheterocyclen der allgemeinen Formel (IB1)

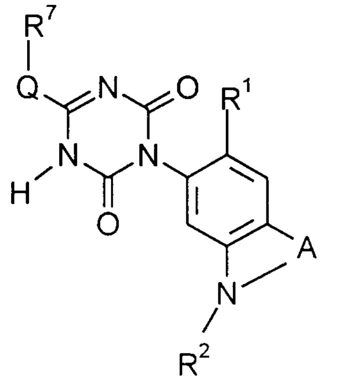

(IB1)

in welcher

$R^1$, $R^2$, $R^7$, A und Q die in Anspruch 1 genannten Bedeutungen haben,

gegebenenfalls in einer anschließenden zweiten Reaktionsstufe mit Alkylierungsmitteln der Formel (III)

$X\text{-}R^{3-1}$ (III)

in welcher

$R^{3-1}$ für gegebenenfalls substituiertes Alkyl steht und

X für Halogen oder die Gruppierung $-O-SO_2-O-R^{3-1}$ steht,

oder mit einem Halogencyan, gegebenenfalls in Gegenwart eines Reaktionshilfsmittels und gegebenenfalls in Gegenwart eines Verdünnungsmittels umsetzt,
und daß man gegebenenfalls die so erhaltenen Verbindungen der Formel (IB) gegebenenfalls in einer dritten Reaktionsstufe nach üblichen Methoden - wie beispielsweise durch thermische Isomerisierung - gegebenenfalls in Gegenwart eines Reaktionshilfmittels und gegebenenfalls in Gegenwart eines Verdünnungsmittels in Verbindungen der Formel (IA) umwandelt.

**5.** Herbizide Mittel, gekennzeichnet durch einen Gehalt an mindestens einem Heterocycylbenzoheterocyclus der Formel (I) gemaß der Ansprüche 1 bis 4.

**6.** Verfahren zur Bekämpfung von unerwünschten Pflanzen, dadurch gekennzeichnet, daß man Heterocyclylbenzoheterocyclen der allgemeinen Formel (I) gemäß den Ansprüchen 1 bis 4 auf Pflanzen und/oder ihren Lebensraum einwirken läßt.

**7.** Verwendung von Heterocyclylbenzoheterocyclen der allgemeinen Formel (I) gemäß der Ansprüche 1 bis 4 zur Bekämpfung von unerwünschten Pflanzen.

**8.** Verfahren zur Herstellung von herbiziden Mitteln, dadurch gekennzeichnet, daß man Heterocyclylbenzoheterocyclen der allgemeinen Formel (I) gemäß der Ansprüche 1 bis 4 mit Streckmitteln und/oder oberflächenaktiven Substanzen vermischt.

**9.** Substituierte Ureidobenzoheterocyclen der allgemeinen Formel (II)

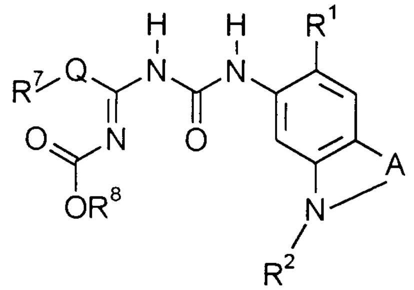

(II)

in welcher

$R^1$ für Wasserstoff oder Halogen steht,

$R^2$ für Wasserstoff, Hydroxy oder für einen jeweils gegebenenfalls substituierten Rest der Reihe Alkyl, Alkenyl, Alkinyl, Cycloalkyl, Cycloalkylalkyl, Alkoxy, Alkenyloxy, Arylalkyl oder Arylalkyloxy steht,

A für eine der nachstehenden Gruppierungen steht
-O-CO-, -S-CO-, -O-C($R^4$,$R^5$)-CO-, -C($R^4$,$R^5$)-O-CO-, -C($R^4$,$R^5$)-CO--O-CO-C-($R^4$,$R^5$)-, -C($R^4$,$R^5$)-C($R^4$,$R^5$)-, -N($R^6$)-C($R^4$,$R^5$)-CO-,

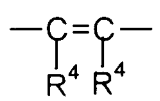 , 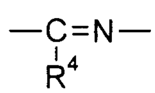

wobei

$R^4$, $R^5$ und $R^6$ gleich oder verschieden sind und einzeln für Wasserstoff oder Alkyl oder zusammen für Alkandiyl stehen und

Q für Sauerstoff oder Schwefel steht,

$R^7$ für Wasserstoff oder für gegebenenfalls substituiertes Alkyl steht und

$R^8$ für Alkyl oder Aryl steht.

**10.** Isocycanatobenzoheterocyclen der allgemeinen Formel (IV)

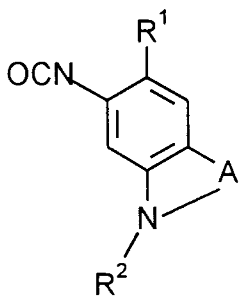

(IV)

in welcher

| | |
|---|---|
| $R^1$ | für Wasserstoff oder Halogen steht, |
| $R^2$ | für Wasserstoff, Hydroxy oder für einen jeweils gegebenenfalls substituierten Rest der Reihe Alkyl, Alkenyl, Alkinyl, Cycloalkyl, Cycloalkylalkyl, Alkoxy, Alkenyloxy, Arylalkyl oder Arylalkyloxy steht, |
| A | für eine der nachstehenden Gruppierungen steht -O-CO-, -S-CO-, -O-C($R^4$,$R^5$)-CO-, -C($R^4$,$R^5$)-O-CO-, -C($R^4$,$R^5$)-CO--O-CO-C-($R^4$,$R^5$)-, -C($R^4$,$R^5$)-C($R^4$,$R^5$)-, -N($R^6$)-C($R^4$,$R^5$)-CO-, |

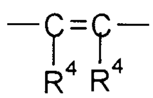 , 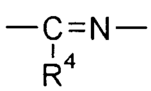

wobei

| | |
|---|---|
| $R^{4,}$ $R^5$ und $R^6$ | gleich oder verschieden sind und einzeln für Wasserstoff oder Alkyl oder zusammen für Alkandiyl stehen. |

Europäisches Patentamt

# EUROPÄISCHER RECHERCHENBERICHT

Nummer der Anmeldung
EP 94 11 2809

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,Y | EP-A-0 170 191 (SUMITOMO CHEMICAL CO., LTD.) 5. Februar 1986<br>* Seite 3, Zeile 9 - Seite 4, Zeile 2; Ansprüche 1-11,16-19 *<br>--- | 1-3,5-8 | C07D413/04<br>C07D265/36<br>C07D279/16 |
| D,Y | EP-A-0 218 972 (SUMITOMO CHEMICAL CO., LTD.) 22. April 1987<br>* Seite 3, Zeile 12 - Seite 4, Zeile 26; Ansprüche 1-7,10-13 *<br>--- | 1-3,5-8 | |
| Y | EP-A-0 248 288 (BAYER AG) 9. Dezember 1987<br>* Seite 3, Zeile 29 - Seite 3, Zeile 34; Ansprüche 1-4,6-9 *<br>--- | 1-3,5-8 | |
| X | EP-A-0 334 135 (BAYER AG) 27. September 1989<br>* Seite 3, Zeile 35 - Seite 3, Zeile 37; Ansprüche 1-10 *<br>----- | 1-3,5-8 | |
| | | | **RECHERCHIERTE SACHGEBIETE (Int.Cl.6)** |
| | | | C07D |

Der vorliegende Recherchenbericht wurde für alle Patentansprüche erstellt

| Recherchenort | Abschlußdatum der Recherche | Prüfer |
|---|---|---|
| MÜNCHEN | 30. November 1994 | Herz, C |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)